# EUROPEAN PATENT SPECIFICATION

(11) **EP 3 135 311 B1**
(45) Date of publication and mention of the grant of the patent: **11.03.2020**
(21) Application number: 16172783.9
(22) Date of filing: 03.06.2016
(51) Int. Cl.: A61L 27/34, A61L 29/04, A61L 29/06, A61L 29/08, A61L 29/14, A61L 31/10, A61L 31/14

(54) **SURFACE MODIFICATION METHOD AND SURFACE-MODIFIED ELASTIC BODY**
OBERFLÄCHENMODIFIZIERUNGSVERFAHREN UND OBERFLÄCHENMODIFIZIERTER ELASTISCHER KÖRPER
PROCÉDÉ DE MODIFICATION DE SURFACE ET CORPS ÉLASTIQUE À SURFACE MODIFIÉE

(30) Priority: 27.08.2015 JP 2015167965
(43) Date of publication of application: 01.03.2017
(73) Proprietor: Sumitomo Rubber Industries, Ltd., Kobe-shi, Hyogo-ken 651-0072 (JP)
(72) Inventor: MINAGAWA, Yasuhisa, Kobe-shi, Hyogo-ken, 651-0072 (JP)
(74) Representative: Manitz Finsterwald Patent- und Rechtsanwaltspartnerschaft mbB

(56) References cited:
- EP-A1- 2 716 669
- EP-A1- 2 796 155
- WO-A1-2014/203668
- WO-A1-2016/042912

## Description

### TECHNICAL FIELD

The present invention relates to surface modification methods capable of providing surfaces which exhibit lubricity when wetted. The present invention also relates to surface-modified elastic bodies, such as surface-modified medical devices or catheters, at least part of whose surface has been modified by the surface modification methods.

### BACKGROUND ART

Catheters used in medical and other fields, such as vascular catheters or urethral catheters for urethral catheterization, are inserted into blood vessels, digestive tracts, tracheae, bile ducts, or ureters and used in aqueous solutions like blood or body fluids. Therefore, they need to be able to be smoothly inserted without damaging tissues.

In order to satisfy this requirement, a low friction lubricant is applied to the surface of a catheter, or the surface of a catheter is coated with a lubricant layer before use (see Patent Literatures 1 to 3). However, unfortunately, these applied or coated lubricant layers have insufficient lubricity and, further, their lubricity is reduced due to e.g. their separation or peeling during movement within a vessel or a tract because these layers are not chemically fixed to the catheter surface.

EP 2 716 669 A1 discloses a method for modifying a surface of an object of a rubber vulcanizate or a thermoplastic elastomer, comprising a step 1 of forming polymerization initiation points on the surface of the object and a step 2 of radical-polymerizing a monomer starting from the polymerization initiation points to grow polymer chains on the surface of the object. A similar method is described in WO 2014/203668 A1 and in EP 2 796 155 A1.

### CITATION LIST

### PATENT LITERATURE

Patent Literature 1: JP 2011-188908 A
Patent Literature 2: JP 2009-518479 T
Patent Literature 3: JP H07-100744 B

### SUMMARY OF INVENTION

### TECHNICAL PROBLEM

The present invention aims to solve the above problems and provide methods for surface-modifying a thermoplastic elastomer. The methods allow these thermoplastic elastomer objects to have a lubricating surface layer chemically fixed thereon and thus exhibit excellent lubricity and excellent lubricant durability, instead of having a resin coating which has drawbacks such as a reduction in lubricity due to e.g. separation or peeling of the coating during movement within a vessel or a tract. Further, the methods have good productivity and good economic efficiency. The present invention also aims to provide surface-modified elastic bodies, such as surface-modified catheters or other medical devices, at least part of whose surface has been modified by the surface modification methods.

### SOLUTION TO PROBLEM

The present invention relates to a method for surface-modifying an object made of nylon, the method including: step 1 of forming polymerization initiation points on a surface of the object; and step 2 of radically polymerizing a monomer starting from the polymerization initiation points by irradiation with ultraviolet light having a wavelength of 300 to 400 nm to grow polymer chains on the surface of the object so that a modification layer having a thickness of 70 to 1,200 nm is formed on the surface, wherein the monomer is a halogen-containing and nitrogen-containing monomer.

The present invention also relates to a method for surface-modifying an object made of nylon, the method including step I of radically polymerizing a monomer by irradiation with ultraviolet light having a wavelength of 300 to 400 nm in the presence of a photopolymerization initiator to grow polymer chains on a surface of the object so that a modification layer having a thickness of 70 to 1,200 nm is formed on the surface, wherein the monomer is a halogen-containing and nitrogen-containing monomer.

The step 1 preferably includes adsorbing a photopolymerization initiator onto a surface of the object, optionally followed by irradiation with ultraviolet light having a wavelength of 300 to 400 nm, to form polymerization initiation points from the photopolymerization initiator on the surface.

The photopolymerization initiator is preferably at least one of a benzophenone compound or a thioxanthone compound.

The method preferably includes introducing an inert gas into a reaction vessel, a reaction pipe, and a reaction solution during or before the light irradiation, and polymerizing the monomer in an atmosphere replaced with the inert gas.

The halogen-containing and nitrogen-containing monomer is preferably at least one of 2-(methacroyloxy)ethyl trimethylammonium chloride or 2-(acryloyloxy)ethyl trimethylammonium chloride.

The present invention relates to a surface-modified elastic body, produced by the surface modification method.

The present invention relates to a surface-modified elastic body, produced by the surface modification method, the elastic body being required to have lubricity in the presence of water.

The present invention relates to a surface-modified elastic body, including a three-dimensional solid body at least part of whose surface is modified by the surface modification method.

The present invention also relates to a catheter, at least part of whose surface is modified by the surface modification method.

### ADVANTAGEOUS EFFECTS OF INVENTION

The methods for surface-modifying an object made of nylon of the present invention include step 1 of forming polymerization initiation points on a surface of the object, and step 2 of radically polymerizing a monomer starting from the polymerization initiation points by irradiation with ultraviolet light having a wavelength of 300 to 400 nm to grow polymer chains on the surface of the object so that a modification layer having a thickness of 70 to 1, 200 nm is formed on the surface; or include step I of radically polymerizing a monomer by irradiation with ultraviolet light having a wavelength of 300 to 400 nm in the presence of a photopolymerization initiator to grow polymer chains on a surface of the object so that a modification layer having a thickness of 70 to 1,200 nm is formed on the surface, wherein the monomer is a halogen-containing and nitrogen-containing monomer.
The objects surface-modified by the methods have a modification layer (polymer chains) of a predetermined thickness having sufficient lubricity fixed to their surface. Thus, the methods of the present invention provide the surface with excellent lubricity and excellent lubricant durability to repeated movements, i.e. a durability such that there will be little reduction in lubricity. Further, the methods are excellent in productivity and economic efficiency. Accordingly, by forming polymer chains on the surface of the objects to be modified according to the methods of the present invention, it is possible to produce surface-modified elastic bodies, such as surface-modified catheters, which are excellent in those properties with good productivity.

### BRIEF DESCRIPTION OF DRAWINGS

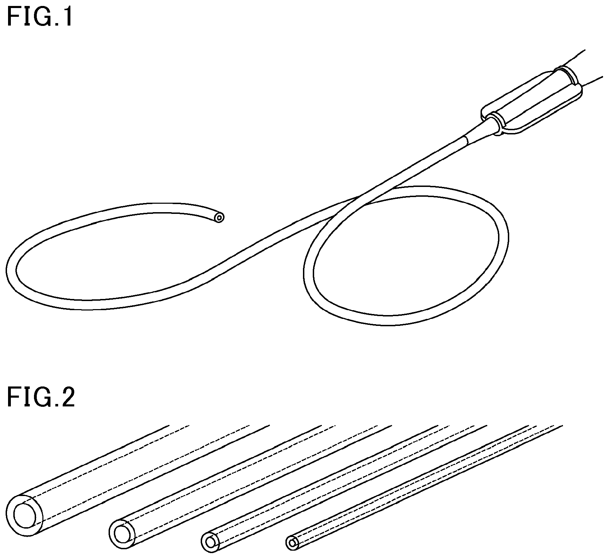

Fig. 1 is an exemplary schematic view of a vascular catheter.
Fig. 2 is an exemplary schematic view showing catheters having different diameters.

### DESCRIPTION OF EMBODIMENTS

The first aspect of the present invention is a method for surface-modifying an object made of nylon, the method including: step 1 of forming polymerization initiation points on a surface of the object; and step 2 of radically polymerizing a monomer starting from the polymerization initiation points by irradiation with ultraviolet light having a wavelength of 300 to 400 nm to grow polymer chains on the surface of the object so that a modification layer having a thickness of 70 to 1,200 nm is formed on the surface, wherein the monomer is a halogen-containing and nitrogen-containing monomer.

By forming particularly a modification layer (modification layer consisting of polymer chains) of a predetermined thickness on a surface of the object by the above method, a surface-modified elastic body can be produced on which polymer chains (modification layer) are fixed to the surface and thus which has excellent lubricity and excellent lubricant durability. Further, since such properties are provided by the modification layer having a thickness of 70 to 1,200 nm according to the method, the method is also excellent in productivity and economic efficiency.

In step 1, polymerization initiation points are formed on the surface of a formed nylon (an object to be modified). For example, the step 1 may be carried out by adsorbing a photopolymerization initiator onto the surface of the object to form polymerization initiation points, or by adsorbing a photopolymerization initiator onto the surface of the object and then irradiating the surface with ultraviolet light having a wavelength of 300 to 400 nm to form polymerization initiation points from the photopolymerization initiator on the surface.

Examples of nylon include nylon 6, nylon 66, nylon 11, and nylon 12.

Examples of the photopolymerization initiator include carbonyl compounds, organic sulfur compounds such as tetraethylthiuram disulfide, persulfides, redox compounds, azo compounds, diazo compounds, halogen compounds, and photoreducing dyes. Preferred among these are carbonyl compounds.

Preferred among carbonyl compounds serving as photopolymerization initiators are benzophenone and derivatives thereof (benzophenone compounds). For example, suitable are benzophenone compounds represented by the following formula: 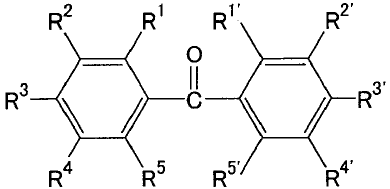 wherein R¹ to R⁵ and R^{1'} to R^{5'} are the same as or different from one another and each represent a hydrogen atom, an alkyl group, a halogen (fluorine, chlorine, bromine, or iodine), a hydroxyl group, a primary to tertiary amino group, a mercapto group, or a hydrocarbon group that may contain an oxygen atom, a nitrogen atom, or a sulfur atom, and any two adjacent groups of R¹ to R⁵ and R^{1'} to R^{5'} may be joined to each other to form a ring together with the carbon atoms to which they are attached.

Specific examples of the benzophenone compound include benzophenone, xanthone, 9-fluorenone, 2,4-dichlorobenzophenone, methyl o-benzoylbenzoate, 4,4'-bis(dimethylamino)benzophenone, and 4,4'-bis(diethylamino)benzophenone. Particularly preferred among these are benzophenone, xanthone, and 9-fluorenone as these compounds contribute to forming polymer brushes well.

The photopolymerization initiator may also suitably be a thioxanthone compound because it provides a high polymerization rate and can easily be adsorbed onto and/or reacted with rubber or the like. Suitable examples include compounds represented by the following formula: 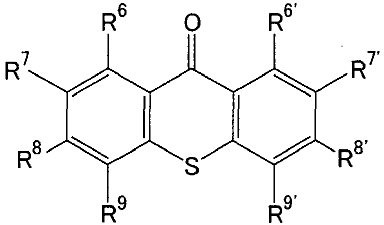 wherein R⁶ to R⁹ and R^{6'} to R^{9'} are the same as or different from one another and each represent a hydrogen atom, a halogen atom, an alkyl group, a cyclic alkyl group, an aryl group, an alkenyl group, an alkoxy group, or an aryloxy group.

Examples of thioxanthone compounds represented by the above formula include thioxanthone, 2-isopropylthioxanthone, 4-isopropylthioxanthone, 2,3-diethylthioxanthone, 2,4-diethylthioxanthone, 2,4-dichlorothioxanthone, 2-methoxythioxanthone, 1-chloro-4-propoxythioxanthone, 2-cyclohexylthioxanthone, 4-cyclohexylthioxanthone, 2-vinylthioxanthone, 2,4-divinylthioxanthone, 2,4-diphenylthioxanthone, 2-butenyl-4-phenylthioxanthone, and 2-p-octyloxyphenyl-4-ethylthioxanthone. Preferred among these are those which are substituted at one or two, especially two, of R⁶ to R⁹ and R^{6'} to R^{9'} with alkyl groups. More preferred is 2,4-diethylthioxanthone.

The adsorption of a photopolymerization initiator such as a benzophenone or thioxanthone compound onto the surface of the object may be carried out as follows. In the case of a benzophenone or thioxanthone compound, for example, the benzophenone or thioxanthone compound is dissolved in an organic solvent to prepare a solution; a surface portion of the object to be modified is treated with this solution so that the compound is adsorbed on the surface portion; and, if necessary, the organic solvent is dried and evaporated off, whereby polymerization initiation points are formed. The surface-treating method may be any method that allows the solution of the benzophenone or thioxanthone compound to be brought into contact with the surface of the object to be modified. Suitable methods include applying or spraying the benzophenone or thioxanthone compound solution onto the surface; or immersing the surface into the solution. When only a part of the surface needs to be modified, it is sufficient to adsorb the photopolymerization initiator only onto the necessary part of the surface. In this case, for example, application or spraying of the solution is suitable. Examples of the organic solvent include methanol, ethanol, acetone, benzene, toluene, methyl ethyl ketone, ethyl acetate, and THF. Acetone is preferred because it does not swell the object intended to be modified, and it dries and evaporates quickly.

As described above, after the photopolymerization initiator is adsorbed on the surface of the object, the surface may further be irradiated with ultraviolet light having a wavelength of 300 to 400 nm to form polymerization initiation points from the photopolymerization initiator on the surface. This ultraviolet light irradiation can be carried out by known methods. For example, it may be carried out in the same manner as described for the ultraviolet light irradiation in step 2, which will be described later.

In step 2, a monomer is radically polymerized starting from the polymerization initiation points formed in step 1, by irradiation with ultraviolet light having a wavelength of 300 to 400 nm to grow polymer chains on the surface of the object so that a modification layer having a thickness of 70 to 1,200 nm is formed on the surface. In particular, by forming a modification layer of a predetermined thickness (a modification layer consisting of polymer chains with a predetermined length) on the surface of the object to be modified, the polymer chains with a predetermined length are sufficiently fixed to the surface, and therefore excellent lubricity and excellent lubricant durability to repeated movements are imparted to the surface with good productivity and good economic efficiency. Herein, the thickness of the modification layer can be measured, for example, using a transmission electron microscope (TEM).

The halogen-containing monomer refers to a monomer containing a halogen atom in the molecule. The halogen-containing monomers may be used alone or in combinations of two or more.

In view of lubricity and lubricant durability, the halogen-containing monomer is according to the present invention a nitrogen-containing monomer (halogen- and nitrogen-containing monomer). Specific preferred examples of such monomers include compounds represented by the following Formula (I): 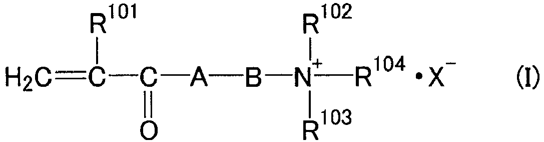 wherein A represents an oxygen atom or NH; B represents a C1-C4 alkylene group; R¹⁰¹ represents a hydrogen atom or a methyl group; R¹⁰², R¹⁰³, and R¹⁰⁴ are the same as or different from one another and each represent a C1-C4 alkyl group; and X⁻ represents a halogen ion.

The symbol A is preferably an oxygen atom. The symbol B may be a linear or branched alkylene group such as a methylene group, an ethylene group, or a propylene group, with a methylene group or an ethylene group being preferred among these. Each of R¹⁰² to R¹⁰⁴ may be a linear or branched alkyl group such as a methyl group, an ethyl group, or a propyl group, with a methyl group or an ethyl group being preferred among these . The symbol X (halogen atom) may be fluorine, chlorine, bromine or the like, preferably chlorine.

Examples of nitrogen-containing monomers represented by Formula (I) include 2-(methacroyloxy)ethyl trimethylammonium chloride (2-(methacroyloxy)ethyl trimethylaminium chloride), 2-(acryloyloxy)ethyl trimethylammonium chloride (2-(acryloyloxy)ethyl trimethylaminium chloride), 2-(methacroyloxy)ethyl dimethylethylammonium chloride (2-(methacroyloxy)ethyl dimethylethylaminium chloride), and 2-(acryloyloxy)ethyl dimethylethylammonium chloride (2-(acryloyloxy)ethyl dimethylethylaminium chloride).

The radical polymerization of a monomer in step 2 may be carried out, for example, as follows: a solution of a monomer or a liquid monomer is applied (sprayed) onto the surface of the object on which a benzophenone or thioxanthone compound or the like has been adsorbed, or the object is immersed in a solution of a monomer or a liquid monomer; and then the object is irradiated with ultraviolet light to allow radical polymerization (photoradical polymerization) of the monomer to proceed, whereby polymer chains are grown on the surface of the object. After the application, the surface of the object may further be covered with a transparent cover of glass, PET, polycarbonate or other materials, followed by irradiating the covered surface with ultraviolet light to allow radical polymerization (photoradical polymerization) of the monomer to proceed, whereby polymer chains are grown on the surface of the object.

The solvent for application (spraying), the method for application (spraying), the method for immersion, the conditions for irradiation, and the like may be conventionally known materials or methods. The solution of the radically polymerizable monomer may be an aqueous solution, or a solution in an organic solvent that does not dissolve the photopolymerization initiator (e.g. benzophenone or thioxanthone compound) used. Moreover, the solution of the radically polymerizable monomer or the liquid radically polymerizable monomer may contain a known polymerization inhibitor such as 4-methylphenol.

In the present invention, the radical polymerization of the monomer is allowed to proceed by light irradiation after the application of the solution of the monomer or the liquid monomer, or after the immersion in the solution of the monomer or the liquid monomer. In the light irradiation, ultraviolet light sources with an emission wavelength mainly in the ultraviolet region, such as high-pressure mercury lamps, metal halide lamps, and LED lamps, can be suitably used. The light dose may be selected appropriately in view of polymerization time and uniform progress of the reaction. Moreover, in order to prevent inhibition of polymerization due to active gas such as oxygen in the reaction vessel and the reaction pipe, oxygen is preferably removed from the reaction vessel, the reaction pipe, and the reaction solution during or before the light irradiation. To this end, appropriate operations may be performed; for example, an inert gas such as nitrogen gas or argon gas is introduced into the reaction vessel, the reaction pipe, and the reaction solution to discharge active gas such as oxygen from the reaction system and replace the atmosphere in the reaction system with the inert gas. Furthermore, in order to prevent inhibition of the reaction due to oxygen and the like, for example, a measure may appropriately be taken in which an ultraviolet light source is placed such that an air layer (oxygen content: 15% or higher) does not exist between the reaction vessel made of glass, plastics or the like and the reaction solution or the object intended to be modified.

The ultraviolet light has a wavelength of 300 to 400 nm. Such a wavelength enables polymer chains to be formed well on the surface of the object. Examples of light sources that can be used include high-pressure mercury lamps, LEDs with a center wavelength of 365 nm, LEDs with a center wavelength of 375 nm, and LEDs with a center wavelength of 385 nm. More preferred is irradiation with LED light having a wavelength of 355 to 390 nm. In particular, for example, LEDs with a center wavelength of 365 nm, which is close to the excitation wavelength (366 nm) of benzophenone, are preferred in view of efficiency. Light having a wavelength of less than 300 nm can cleave and damage molecules of the object intended to be modified. For this reason, light having a wavelength of 300 nm or greater is preferred. More preferred is light having a wavelength of 355 nm or greater because it produces very little damage to the object. In contrast, light having a wavelength of greater than 400 nm is less likely to activate the polymerization initiator, so that the polymerization reaction does not readily proceed. For this reason, light having a wavelength of 400 nm or less is preferred. Although LED light is suitable because the wavelength range of LED light is narrow so that no wavelengths other than the center wavelength are emitted, a mercury lamp or the like can also achieve similar effects to those of LED light if a filter is used to block light with wavelengths less than 300 nm.

In step 2, the radical polymerization is performed to grow polymer chains on the surface of the object so that a modification layer having a thickness of 70 to 1, 200 nm is formed on the surface. The modification layer with the above range thus formed provides excellent lubricity and excellent lubricant durability to repeated movements. The modification layer preferably has a thickness of 80 to 1,100 nm. The modification layer (modification layer consisting of polymer chains) of a predetermined thickness can be formed by appropriately choosing or controlling, for example, the duration of ultraviolet light irradiation, the wavelength of ultraviolet light, the amount of the photopolymerization initiator used, the type and concentration of the monomer, polymerization temperature, the addition of a catalyst, and other conditions.

The duration of irradiation with light having a wavelength of 300 to 400 nm may be selected appropriately so that the modification layer of a predetermined thickness is formed. For example, the irradiation is carried out for 30 to 500 minutes.

The second aspect of the present invention is a method for surface-modifying an object made of nylon, the method including step I of radically polymerizing a monomer by irradiation with ultraviolet light having a wavelength of 300 to 400 nm in the presence of a photopolymerization initiator to grow polymer chains on a surface of the object so that a modification layer having a thickness of 70 to 1,200 nm is formed on the surface. Specifically, by radically polymerizing a monomer by irradiation with ultraviolet light in the presence of a photopolymerization initiator as a polymerization initiator to form particularly a modification layer (modification layer consisting of polymer chains) of a predetermined thickness on the surface of the object, a surface-modified elastic body can be produced on which polymer chains (modification layer) are fixed to the surface and thus which has excellent lubricity and excellent lubricant durability. Further, since such properties are provided by the modification layer having a thickness of 70 to 1,200 nm according to the method, the method is also excellent in productivity and economic efficiency. The object to be modified, the photopolymerization initiator, and the monomer used in step I may be the same as those described above.

For example, step I may be carried out as follows: a photopolymerization initiator and a monomer are brought into contact with the surface of the object to be modified, and then the surface is irradiated with LED light having a wavelength of 300 to 400 nm to form polymerization initiation points from the photopolymerization initiator while simultaneously radically polymerizing the monomer starting from the polymerization initiation points to grow polymer chains so that a modification layer having a thickness of 70 to 1,200 nm is formed on the surface.

The radical polymerization of a monomer in step I may be carried out as follows: a solution of a monomer or a liquid monomer which contains a photopolymerization initiator such as a benzophenone or thioxanthone compound is applied (sprayed) onto the surface of the object to be modified, or the object is immersed in a solution of a monomer or a liquid monomer which contains a photopolymerization initiator; and then the object is irradiated with ultraviolet light to allow radical polymerization (photoradical polymerization) of the monomer to proceed, whereby polymer chains are grown on the surface of the object so that a modification layer of a predetermined thickness is formed. Further, the surface of the object may be covered with a transparent cover of glass, PET, polycarbonate or other materials, followed by irradiating the covered surface with ultraviolet light as described hereinabove. The solvent for application (spraying), the method for application (spraying), the method for immersion, the conditions for irradiation, and the like may be the materials or methods described hereinabove.

In step I, the radical polymerization is performed to grow polymer chains on the surface of the object so that a modification layer having a thickness of 70 to 1,200 nm is formed on the surface. The modification layer with the above range thus formed provides excellent lubricity and excellent lubricant durability to repeated movements. The modification layer preferably has a thickness of 80 to 1,100 nm. The modification layer (modification layer consisting of polymer chains) of a predetermined thickness can be formed by appropriately choosing or controlling, for example, the duration of ultraviolet light irradiation, the wavelength of ultraviolet light, the amount of the photopolymerization initiator used, the type and concentration of the monomer, polymerization temperature, the addition of a catalyst, and other conditions.

Similarly to the above-described embodiments, the duration of irradiation with light having a wavelength of 300 to 400 nm may be selected appropriately so that a modification layer of a predetermined thickness is formed. For example, the irradiation is carried out for 30 to 500 minutes.

In step 2 or step I, two or more types of monomers may be radically polymerized simultaneously. Moreover, multiple types of polymer chains may be grown on the surface of the object to be modified. In the surface modification methods of the present invention, the polymer chains may be crosslinked to one another. In this case, the polymer chains may be crosslinked to one another by ionic crosslinking, crosslinking by a hydrophilic group containing an oxygen atom, or crosslinking by a halogen group such as iodine.

The above-described surface modification methods can be applied to nylon to produce surface-modified elastic bodies, for example surface-modified elastic bodies that are excellent in lubricity in the presence of water. The methods may also be applied to at least a part of a three-dimensional solid body (e.g. elastic body) to produce a surface-modified elastic body with modified properties. Furthermore, preferred examples of the surface-modified elastic body include polymer brushes. The term "polymer brush" means an assembly of graft polymer molecules obtained in the "grafting from" approach by surface-initiated living radical polymerization. The graft chains are preferably oriented in a direction substantially vertical to the surface of the object because then the entropy decreases to reduce the molecular mobility of the graft chains, thereby providing lubricity. Furthermore, semidilute or concentrated brushes having a brush density of 0.01 chains/nm² or higher are preferred.

The surface modification methods may also be applied to nylon to produce medical devices, such as catheters, at least part of whose surface is modified. The modification is applied to the surface of medical devices such as catheters preferably at least at a portion that requires lubricity, and may be applied to the entire surface.

### EXAMPLES

The present invention is more specifically described with reference to, but not limited to, examples below.

### (Example 1)

A 3 wt% solution of benzophenone in acetone was applied to the surface of a thermoplastic elastomer tube made of nylon 12 so that benzophenone was adsorbed onto the surface, followed by drying.

Subsequently, the tube was immersed in an aqueous solution of 2- (methacroyloxy) ethyl trimethylammonium chloride (1.25 M) in a glass reaction vessel. The reaction vessel was sealed with a rubber stopper, and argon gas was introduced and allowed to bubble through the solution for 120 minutes to remove oxygen. The glass reaction vessel was irradiated with LED light (5 mW/cm²) having a wavelength of 365 nm for 180 minutes while being rotated. Thus, radical polymerization was carried out to grow polymer chains on the surface of the nylon tube, whereby a surface-modified elastic body (polymer brush) was prepared.

### (Example 2)

An aqueous solution of 2-(methacroyloxy)ethyl trimethylammonium chloride (1.25 M) to which benzophenone was added and adjusted at a concentration of 0.1% by mass was prepared and put in a glass reaction vessel. The surface of a thermoplastic elastomer tube made of nylon 12 was immersed in this aqueous solution. The reaction vessel was sealed with a rubber stopper, and argon gas was introduced and allowed to bubble through the solution for 120 minutes to remove oxygen. The glass reaction vessel was irradiated with LED light (5 mW/cm²) having a wavelength of 365 nm for 180 minutes while being rotated. Thus, radical polymerization was carried out to grow polymer chains on the surface of the nylon tube, whereby a surface-modified elastic body (polymer brush) was prepared.

### (Example 3)

A 3 wt% solution of benzophenone in acetone was applied to the surface of a thermoplastic elastomer tube made of nylon 12 so that benzophenone was adsorbed onto the surface, followed by drying.

Subsequently, the tube was immersed in an aqueous solution of 2- (methacroyloxy) ethyl trimethylammonium chloride (1.25 M) in a glass reaction vessel. The reaction vessel was sealed with a rubber stopper, and argon gas was introduced and allowed to bubble through the solution for 120 minutes to remove oxygen. The glass reaction vessel was irradiated with LED light (5 mW/cm²) having a wavelength of 365 nm for 120 minutes while being rotated. Thus, radical polymerization was carried out to grow polymer chains on the surface of the nylon tube, whereby a surface-modified elastic body (polymer brush) was prepared.

### (Example 4) (not according to the present invention)

A 3 wt% solution of benzophenone in acetone was applied to the surface of a thermoplastic elastomer tube made of nylon 12 so that benzophenone was adsorbed onto the surface, followed by drying.

Subsequently, the tube was immersed in an aqueous solution of potassium 3-sulfopropyl methacrylate (1.25 M) in a glass reaction vessel. The reaction vessel was sealed with a rubber stopper, and argon gas was introduced and allowed to bubble through the solution for 120 minutes to remove oxygen. The glass reaction vessel was irradiated with LED light (5 mW/cm²) having a wavelength of 365 nm for 200 minutes while being rotated. Thus, radical polymerization was carried out to grow polymer chains on the surface of the nylon tube, whereby a surface-modified elastic body (polymer brush) was prepared.

### (Example 5) (not according to the present invention)

A 3 wt% solution of benzophenone in acetone was applied to the surface of a thermoplastic elastomer tube made of nylon 12 so that benzophenone was adsorbed onto the surface, followed by drying.

Subsequently, the tube was immersed in an aqueous solution of potassium 3-sulfopropyl methacrylate (1.25 M) in a glass reaction vessel. The reaction vessel was sealed with a rubber stopper, and argon gas was introduced and allowed to bubble through the solution for 120 minutes to remove oxygen. The glass reaction vessel was irradiated with LED light (5 mW/cm²) having a wavelength of 365 nm for 300 minutes while being rotated. Thus, radical polymerization was carried out to grow polymer chains on the surface of the nylon tube, whereby a surface-modified elastic body (polymer brush) was prepared.

### (Example 6) (not according to the present invention)

A 3 wt% solution of benzophenone in acetone was applied to the surface of a thermoplastic elastomer tube made of nylon 12 so that benzophenone was adsorbed onto the surface, followed by drying.

Subsequently, the tube was immersed in an aqueous solution of 2-methacryloyloxyethyl phosphorylcholine (1.25 M) in a glass reaction vessel. The reaction vessel was sealed with a rubber stopper, and argon gas was introduced and allowed to bubble through the solution for 120 minutes to remove oxygen. The glass reaction vessel was irradiated with LED light (5 mW/cm²) having a wavelength of 365 nm for 210 minutes while being rotated. Thus, radical polymerization was carried out to grow polymer chains on the surface of the nylon tube, whereby a surface-modified elastic body (polymer brush) was prepared.

### (Example 7) (not according to the present invention)

A 3 wt% solution of benzophenone in acetone was applied to the surface of a thermoplastic elastomer tube made of nylon 12 so that benzophenone was adsorbed onto the surface, followed by drying.

Subsequently, the tube was immersed in an aqueous solution of 2-methacryloyloxyethyl phosphorylcholine (1.25 M) in a glass reaction vessel. The reaction vessel was sealed with a rubber stopper, and argon gas was introduced and allowed to bubble through the solution for 120 minutes to remove oxygen. The glass reaction vessel was irradiated with LED light (5 mW/cm²) having a wavelength of 365 nm for 300 minutes while being rotated. Thus, radical polymerization was carried out to grow polymer chains on the surface of the nylon tube, whereby a surface-modified elastic body (polymer brush) was prepared.

### (Example 8)

A 3 wt% solution of 2,4-diethylthioxanthone in acetone was applied to the surface of a thermoplastic elastomer tube made of nylon 12 so that 2,4-diethylthioxanthone was adsorbed onto the surface, followed by drying.

Subsequently, the tube was immersed in an aqueous solution of 2-(methacroyloxy) ethyl trimethylammonium chloride (1.25 M) in a glass reaction vessel. The reaction vessel was sealed with a rubber stopper, and argon gas was introduced and allowed to bubble through the solution for 120 minutes to remove oxygen. The glass reaction vessel was irradiated with LED light (5 mW/cm²) having a wavelength of 365 nm for 90 minutes while being rotated. Thus, radical polymerization was carried out to grow polymer chains on the surface of the nylon tube, whereby a surface-modified elastic body (polymer brush) was prepared.

### (Example 9)

A 3 wt% solution of 2,4-diethylthioxanthone in acetone was applied to the surface of a thermoplastic elastomer tube made of nylon 12 so that 2,4-diethylthioxanthone was adsorbed onto the surface, followed by drying.

Subsequently, the tube was immersed in an aqueous solution of 2-(methacroyloxy)ethyl trimethylammonium chloride (1.25 M) in a glass reaction vessel. The reaction vessel was sealed with a rubber stopper, and argon gas was introduced and allowed to bubble through the solution for 120 minutes to remove oxygen. The glass reaction vessel was irradiated with LED light (5 mW/cm²) having a wavelength of 385 nm for 60 minutes while being rotated. Thus, radical polymerization was carried out to grow polymer chains on the surface of the nylon tube, whereby a surface-modified elastic body (polymer brush) was prepared.

### (Example 10) (not according to the present invention)

A surface-modified elastic body (a polymer brush in which polymer chains were grown on the surface of a polyurethane tube) was prepared as in Example 1, except that a polyurethane tube was used instead of the nylon tube.

### (Example 11)

A 3 wt% solution of benzophenone in acetone was applied to the surface of a thermoplastic elastomer tube made of nylon 12 so that benzophenone was adsorbed onto the surface, followed by drying.

Subsequently, the tube was immersed in an aqueous solution of 2-(methacroyloxy)ethyl trimethylammonium chloride (1.25 M) in a glass reaction vessel. The reaction vessel was sealed with a rubber stopper, and argon gas was introduced and allowed to bubble through the solution for 120 minutes to remove oxygen. The glass reaction vessel was irradiated with LED light (5 mW/cm²) having a wavelength of 365 nm for 120 minutes while being rotated and warmed with a heater at about 50°C. Thus, radical polymerization was carried out to grow polymer chains on the surface of the nylon tube, whereby a surface-modified elastic body (polymer brush) was prepared.

### (Comparative Example 1)

A tube made of nylon 12 was used as it was.

### (Comparative Example 2)

The surface of a tube made of nylon 12 was coated with a 5% solution of methyl vinyl ether-maleic anhydride (GANTREZ-AN 16, available from IPS) in methanol, and this coated tube was used. It should be noted that nylon 12 is a material often used for vascular catheters, and methyl vinyl ether-maleic anhydride is a typical lubricant to impart lubricity to surfaces.

### (Comparative Example 3)

A 3 wt% solution of benzophenone in acetone was applied to the surface of a thermoplastic elastomer tube made of nylon 12 so that benzophenone was adsorbed onto the surface, followed by drying.

Subsequently, the tube was immersed in an aqueous solution of potassium 3-sulfopropyl methacrylate (1.25 M) in a glass reaction vessel. The reaction vessel was sealed with a rubber stopper, and argon gas was introduced and allowed to bubble through the solution for 120 minutes to remove oxygen. The glass reaction vessel was irradiated with LED light (5 mW/cm²) having a wavelength of 365 nm for 120 minutes while being rotated. Thus, radical polymerization was carried out to grow polymer chains on the surface of the nylon tube, whereby a surface-modified elastic body (polymer brush) was prepared.

### (Comparative Example 4)

A 3 wt% solution of benzophenone in acetone was applied to the surface of a thermoplastic elastomer tube made of nylon 12 so that benzophenone was adsorbed onto the surface, followed by drying.

Subsequently, the tube was immersed in an aqueous solution of 2-methacryloyloxyethyl phosphorylcholine (1.25 M) in a glass reaction vessel. The reaction vessel was sealed with a rubber stopper, and argon gas was introduced and allowed to bubble through the solution for 120 minutes to remove oxygen. The glass reaction vessel was irradiated with LED light (5 mW/cm²) having a wavelength of 365 nm for 150 minutes while being rotated. Thus, radical polymerization was carried out to grow polymer chains on the surface of the nylon tube, whereby a surface-modified elastic body (polymer brush) was prepared.

### (Comparative Example 5)

A 3 wt% solution of benzophenone in acetone was applied to the surface of a thermoplastic elastomer tube made of nylon 12 so that benzophenone was adsorbed onto the surface, followed by drying.

Subsequently, the tube was immersed in an aqueous solution of 2-(methacroyloxy)ethyl trimethylammonium chloride (1.25 M) in a glass reaction vessel. The reaction vessel was sealed with a rubber stopper, and argon gas was introduced and allowed to bubble through the solution for 120 minutes to remove oxygen. The glass reaction vessel was irradiated with LED light (5 mW/cm²) having a wavelength of 365 nm for 300 minutes while being rotated. Thus, radical polymerization was carried out to grow polymer chains on the surface of the nylon tube, whereby a surface-modified elastic body (polymer brush) was prepared.

The irradiation with LED light having a wavelength of 365 nm was carried out at room temperature, except for Example 11.

The surface-modified elastic bodies prepared in the examples and the comparative examples were evaluated as follows.

### (Thickness of graft layer (modification layer))

The surface of the tube was stained with osmic acid. A cross section prepared from the stained tube by frozen ultra-thin sectioning was observed using a transmission electron microscope (TEM) at 100 kV to determine the thickness of the graft layer (thickness of the modification layer consisting of polymer chains).

### (Lubricity)

Water was applied to the surface of the tube, and the sliding properties of the surface were subjectively evaluated by touching with a human finger. The subjective evaluation was carried out by ten persons according to a rating scale of 1-5, where a rating of 5 corresponds to a tube with good sliding properties, and a rating of 1 corresponds to a tube with poor sliding properties that do not allow the finger to slide on the surface. The average of the ratings was calculated.

### (Lubricant durability (Durability, Rate of decrease))

After water was applied to the surface of the tube, the tube was held between fingers and slid on the fingers. This cycle was repeated 100 times. Then, the subjective evaluation was again carried out by ten persons according to the rating scale for lubricity, and the average of the ratings and the rate of decrease from the initial lubricity were calculated.

**[Table 1]**

| | Example | | | | | | | | | | |
|---|---|---|---|---|---|---|---|---|---|---|---|
| | 1 | 2 | 3 | 4 | 5 | 6 | 7 | 8 | 9 | 10 | 11 |
| Thickness (nm) of graft layer (modification layer) | 1000 | 780 | 650 | 100 | 150 | 120 | 180 | 880 | 920 | 780 | 890 |
| Lubricity | 4.9 | 4.9 | 4.9 | 4.6 | 4.8 | 4.7 | 4.8 | 4.9 | 4.9 | 4.4 | 4.9 |
| Durability | 4.8 | 4.8 | 4.8 | 4.5 | 4.7 | 4.6 | 4.7 | 4.8 | 4.8 | 4.2 | 4.8 |
| Rate of decrease | 2.0% | 2.0% | 2.0% | 2.2% | 2.1% | 2.1% | 2.1% | 2.0% | 2.0% | 4.5% | 2.0% |

### Examples 4 to 7 and 10 are not according to the present invention

**[Table 2]**

| | Comparative Example | | | | |
|---|---|---|---|---|---|
| | 1 | 2 | 3 | 4 | 5 |
| Thickness (nm) of graft layer (modification layer) | - | - | 60 | 55 | 1500 |
| Lubricity | 1 | 4.2 | 1.7 | 1.9 | 4.9 |
| Durability | 1 | 2.4 | 1.4 | 1.6 | 4.8 |
| Rate of decrease | 0% | 43% | 17.6% | 15.8% | 2.0% |

As shown in Tables 1 and 2, comparisons between Examples 4 and 5 and Comparative Example 3 and between Examples 6 and 7 and Comparative Example 4 reveal that graft layers having a thickness of less than 70 nm gave very poor lubricity, whereas graft layers having a thickness of more than 70 nm gave high lubricity and sufficient durability. Moreover, the polyurethane tube (Example 10, not according to the invention) as well as the nylon tubes exhibited sufficient lubricity and sufficient lubricant durability.

In contrast, the untreated tube of Comparative Example 1 exhibited very poor lubricity, and the commonly used product of Comparative Example 2 had moderately high initial lubricity but exhibited low durability and quite a high rate of decrease in lubricity. The tube of Comparative Example 5 with a thickness of more than 1,200 nm exhibited almost the same levels of lubricity and durability as the tube of Example 1, but the productivity and economic efficiency of Comparative Example 5 were inferior due to the long polymerization time. Moreover, the thick graft layer of Comparative Example 5 was disadvantageously accompanied by a large amount of free polymers which were not fixed to the substrate.

The above results demonstrated that sufficient lubricity and sufficient lubricant durability can be simultaneously imparted to the surface of vascular catheters or other objects by forming polymer chains on the surface from a monomer such as potassium 3-sulfopropyl methacrylate (not according to the invention), 2-methacryloyloxyethyl phosphorylcholine (not according to the invention), or 2-(methacroyloxy)ethyl trimethylammonium chloride.

## Claims

1. A method for surface-modifying an object made of nylon, the method comprising:
step 1 of forming polymerization initiation points on a surface of the object; and
step 2 of radically polymerizing a monomer starting from the polymerization initiation points by irradiation with ultraviolet light having a wavelength of 300 to 400 nm to grow polymer chains on the surface of the object so that a modification layer having a thickness of 70 to 1,200 nm is formed on the surface, wherein the monomer is a halogen-containing and nitrogen-containing monomer.

2. A method for surface-modifying an object made of nylon, the method comprising
step I of radically polymerizing a monomer by irradiation with ultraviolet light having a wavelength of 300 to 400 nm in the presence of a photopolymerization initiator to grow polymer chains on a surface of the object so that a modification layer having a thickness of 70 to 1, 200 nm is formed on the surface, wherein the monomer is a halogen-containing and nitrogen-containing monomer.

3. The method according to claim 1,
wherein the step 1 comprises adsorbing a photopolymerization initiator onto a surface of the object, optionally followed by irradiation with ultraviolet light having a wavelength of 300 to 400 nm, to form polymerization initiation points from the photopolymerization initiator on the surface.

4. The method according to claim 2 or 3,
wherein the photopolymerization initiator is at least one of a benzophenone compound or a thioxanthone compound.

5. The method according to any one of claims 1 to 4,
wherein the method comprises introducing an inert gas into a reaction vessel, a reaction pipe, and a reaction solution during or before the light irradiation, and polymerizing the monomer in an atmosphere replaced with the inert gas.

6. The method according to claim 1 or 2,
wherein the halogen-containing and nitrogen-containing monomer is at least one of 2-(methacroyloxy)ethyl trimethylammonium chloride or 2-(acryloyloxy)ethyl trimethylammonium chloride.

7. The method according to any one of claims 1 to 6,
wherein a solution of the monomer, or the monomer in the liquid state contains a polymerization inhibitor, and is polymerized in the presence of the polymerization inhibitor.

8. A surface-modified elastic body, produced by the method according to any one of claims 1 to 7.

9. A surface-modified elastic body, according to claim 8, wherein the elastic body is required to have lubricity in the presence of water.

10. A surface-modified elastic body according to claim 8, comprising
a three-dimensional solid body at least part of whose surface is modified by the method according to any one of claims 1 to 7.

11. A surface-modified elastic body according to claim 8, wherein the surface-modified elastic body is a catheter, at least part of whose surface is modified by the method according to any one of claims 1 to 7.

## Patentansprüche

1. Verfahren zur Oberflächenmodifizierung eines aus Nylon hergestellten Gegenstands, wobei das Verfahren umfasst:
Schritt 1 der Bildung von Polymerisations-Initiationspunkten auf einer Oberfläche des Gegenstands; und
Schritt 2 der radikalischen Polymerisierung eines Monomers, ausgehend von den Polymerisations-Initiationspunkten, durch Bestrahlung mit ultraviolettem Licht mit einer Wellenlänge von 300 bis 400 nm, um auf der Oberfläche des Gegenstands Polymerketten zu erzeugen, so dass auf der Oberfläche des Gegenstands eine Modifikationsschicht mit einer Dicke von 70 bis 1200 nm gebildet wird, wobei das Monomer ein halogenhaltiges und stickstoffhaltiges Monomer ist.

2. Verfahren zur Oberflächenmodifizierung eines aus Nylon hergestellten Gegenstands, wobei das Verfahren umfasst:
Schritt I der radikalischen Polymerisierung eines Monomers durch Bestrahlung mit ultraviolettem Licht mit einer Wellenlänge von 300 bis 400 nm in der Gegenwart eines Photopolymerisations-Initiatiors, um auf einer Oberfläche des Gegenstands Polymerketten zu erzeugen, sodass auf der Oberfläche eine Modifikationsschicht mit einer Dicke von 70 bis 1200 nm gebildet wird, wobei das Monomer ein halogenhaltiges und stickstoffhaltiges Monomer ist.

3. Verfahren nach Anspruch 1,
wobei der Schritt 1 das Adsorbieren eines Photopolymerisations-Initiators auf einer Oberfläche des Gegenstands umfasst, optional gefolgt von Bestrahlung mit ultraviolettem Licht mit einer Wellenlänge von 300 bis 400 nm, um aus dem Photopolymerisations-Initiator auf der Oberfläche Polymerisations-Initiationspunkte zu bilden.

4. Verfahren nach Anspruch 2 oder 3,
wobei der Photopolymerisations-Initiator zumindest eine von einer Benzophenon-Verbindung oder einer Thioxanthon-Verbindung ist.

5. Verfahren nach einem der Ansprüche 1 bis 4,
wobei das Verfahren umfasst, dass während oder vor der Bestrahlung mit Licht ein Inertgas in ein Reaktionsgefäß, ein Reaktionsrohr und eine Reaktionslösung eingeleitet wird, und das Monomer in einer durch das Inertgas verdrängten Atmosphäre polymerisiert wird.

6. Verfahren nach Anspruch 1 oder 2,
wobei das halogenhaltige und stickstoffhaltige Monomer mindestens eines von 2-(Methacroyloxy)ethyltrimethylammoniumchlorid oder 2-(Acryloyloxy)ethyltrimethylammoniumchlorid ist.

7. Verfahren nach einem der Ansprüche 1 bis 6,
wobei eine Lösung des Monomers, oder das Monomer im flüssigen Zustand, einen Polymerisitationsinhibitor enthält und in der Gegenwart des Polymerisationsinhibitors polymerisiert wird.

8. Oberflächenmodifizierter elastischer Körper, hergestellt durch das Verfahren nach einem der Ansprüche 1 bis 7.

9. Oberflächenmodifizierter elastischer Körper nach Anspruch 8, wobei von dem elastischen Körper Gleitfähigkeit in der Gegenwart von Wasser gefordert ist.

10. Oberflächenmodifizierter elastischer Körper nach Anspruch 8, welcher umfasst:
einen dreidimensionalen festen Körper, dessen Oberfläche zumindest teilweise durch das Verfahren nach einem der Ansprüche 1 bis 7 modifiziert ist.

11. Oberflächenmodifizierter elastischer Körper nach Anspruch 8, wobei der oberflächenmodifizierte elastische Körper ein Katheter ist, dessen Oberfläche zumindest teilweise durch das Verfahren nach einem der Ansprüche 1 bis 7 modifiziert ist.

## Revendications

1. Procédé de modification de surface d'un objet constitué de nylon, le procédé comprenant :
l'étape 1 de formation de points d'initiation de polymérisation sur une surface de l'objet ; et
l'étape 2 de polymérisation radicale d'un monomère en commençant à partir des points d'initiation de polymérisation par irradiation avec de la lumière ultraviolette ayant une longueur d'onde de 300 à 400 nm pour faire croître les chaînes de polymère sur la surface de l'objet de sorte qu'une couche de modification ayant une épaisseur de 70 à 1200 nm est formée sur la surface, dans lequel le monomère est un monomère contenant un halogène et contenant un azote.

2. Procédé de modification de surface d'un objet constitué de nylon, le procédé comprenant :
l'étape I de polymérisation radicale d'un monomère par irradiation avec de la lumière ultraviolette ayant une longueur d'onde de 300 à 400 nm en présence d'un initiateur de photopolymérisation pour faire croître les chaînes de polymère sur une surface de l'objet de sorte qu'une couche de modification ayant une épaisseur de 70 à 1200 nm est formée sur la surface, dans lequel le monomère est un monomère contenant un halogène et contenant un azote.

3. Procédé selon la revendication 1,
dans lequel l'étape 1 comprend l'adsorption d'un initiateur de photopolymérisation sur une surface de l'objet, éventuellement suivie par une irradiation avec de la lumière ultraviolette ayant une longueur d'onde de 300 à 400 nm, pour former des points d'initiation de polymérisation à partir de l'initiateur de photopolymérisation sur la surface.

4. Procédé selon la revendication 2 ou 3, dans lequel
l'initiateur de photoplymérisation est au moins un d'un composé benzophénone ou d'un composé thioxanthone.

5. Procédé selon l'une quelconque des revendications 1 à 4,
dans lequel le procédé comprend l'introduction d'un gaz inerte dans un récipient de réaction, un tuyau de réaction et une solution de réaction durant ou avant l'irradiation par la lumière, et la polymérisation du monomère dans une atmosphère remplacée avec le gaz inerte.

6. Procédé selon la revendication 1 ou 2,
dans lequel le monomère contenant un halogène et contenant un azote est au moins un du chlorure de 2-(méthacroyloxy)éthyl triméthylammonium ou du chlorure de 2-(acryloyloxy)éthyl triméthylammonium.

7. Procédé selon l'une quelconque des revendications 1 à 6,
dans lequel une solution du monomère, ou le monomère dans l'état liquide contient un inhibiteur de polymérisation, et est polymérisé en présence de l'inhibiteur de polymérisation.

8. Corps élastique modifié en surface, produit par le procédé selon l'une quelconque des revendications 1 à 7.

9. Corps élastique modifié en surface, selon la revendication 8, lequel corps élastique est requis pour avoir une lubricité en présence d'eau.

10. Corps élastique modifié en surface selon la revendication 8, comprenant : un corps solide tri-dimensionnel dont au moins une partie de la surface est modifiée par le procédé selon l'une quelconque des revendications 1 à 7.

11. Corps élastique modifié en surface selon la revendication 8, dans lequel le corps élastique modifié en surface est un cathéter, dont au moins une partie de la surface est modifiée selon l'une quelconque des revendications 1 à 7.
